(19) Europäisches Patentamt
European Patent Office
Office européen des brevets

(11) EP 0 887 069 B1

(12) EUROPÄISCHE PATENTSCHRIFT

(45) Veröffentlichungstag und Bekanntmachung des Hinweises auf die Patenterteilung:
**24.03.2004 Patentblatt 2004/13**

(51) Int Cl.$^7$: **A61K 7/42**, A61K 7/48

(21) Anmeldenummer: **98110763.4**

(22) Anmeldetag: **12.06.1998**

# (54) Verwendung von synthetischem Bienenwachs zur Verstärkung des Lichtschutzfaktors kosmetischer oder dermatologischer Lichtschutzmittel

Use of synthetic beeswax to increase the protection factor of cosmetic or dermatological sunscreens

Utilisation de cire d'abeilles synthétique pour augmenter l'indice de protection des compositions cosmétiques et dermatologiques photoprotectrices

(84) Benannte Vertragsstaaten:
**AT BE CH DE ES FR GB IT LI NL SE**

(30) Priorität: **24.06.1997 DE 19726786**

(43) Veröffentlichungstag der Anmeldung:
**30.12.1998 Patentblatt 1998/53**

(73) Patentinhaber: **Beiersdorf Aktiengesellschaft
20245 Hamburg (DE)**

(72) Erfinder:
- **Gers-Barlag, Heinrich, Dr.
25495 Kummerfeld (DE)**
- **Kröpke, Rainer
22527 Hamburg (DE)**
- **von der Fecht, Stephanie
22869 Schenefeld (DE)**
- **Müller, Anja
23843 Rümpel (DE)**

(56) Entgegenhaltungen:
**DE-A- 2 241 261**
**US-A- 3 914 131**
**US-A- 4 761 276**

EP 0 887 069 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft Lichtschutzformulierungen, insbesondere kosmetische und dermatologische Lichtschutzmittel.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig. Filtersubsubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altem läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)) nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen. Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Um diesen Reaktionen vorzubeugen, können den kosmetischen bzw. dermatologischen Formulierungen zusätzlich Antioxidantien und/oder Radikalfänger einverleibt werden.

Die teilweise vorstehend genannten Verbindungen, welche als Lichtschutzmittel für kosmetische und dermatologische Lichtschutzformulierungen eingesetzt werden, zeichnen sich an sich durch gute Lichtschutzwirkung aus. Sie haben jedoch den Nachteil, daß es bisweilen schwierig ist, sie in befriedigender Weise solchen Formulierungen einzuverleiben. Ferner sind diese Verbindungen reine UV-Absorber und als Radikalfänger ungeeignet.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Für die Dosierung der Substanzen in den fertigen Formulierungen können die Extinktionswerte allenfalls eine Orientierungshilfe bieten, denn durch Wechselwirkungen mit Inhaltsstoffen der Haut oder der Hautoberfläche selbst können Unwägbarkeiten auftreten. Ferner ist in der Regel schwierig vorab abzuschätzen, wie gleichmäßig und in welcher Schichtdicke die Filtersubstanz in und auf der Homschicht der Haut verteilt ist.

Der Lichtschutzfaktor (LSF, oft auch, dem englischen Sprachgebrauch angepaßt, SPF genannt) gibt an, um wieviel länger die mit dem Lichtschutzmittel geschützte Haut bestrahlt werden kann, bis die gleiche Erythemreaktion auftritt wie bei der ungeschützten Haut (also zehnmal solang gegenüber ungeschützter Haut bei LSF = 10).

Jedenfalls erwartet der Verbraucher zum einen - nicht zuletzt wegen der ins Licht der Öffentlichkeit gerückten Diskussion über das sogenannte "Ozonloch" zum einen zuverlässige Angaben des Herstellers zum Lichtschutzfaktor, zum anderen geht eine Tendenz des Verbrauchers zu höheren und hohen Lichtschutzfaktoren.

Da Lichtschutzfiltersubstanzen in der Regel kostspielig sind, und da manche Lichtschutzfiltersubstanzen zudem schwierig in höheren Konzentrationen in kosmetische oder dermatologische Zubereitungen einzuarbeiten sind, war es Aufgabe der Erfindung, auf einfache und preiswerte Weise zu Zubereitungen zu gelangen, welche bei ungewöhnlich niedrigen Konzentrationen an herkömmlichen Lichtschutzfiltersubstanzen dennoch akzeptable oder sogar hohe LSF-Werte erreichen.

Es war indes überraschend und für den Fachmann nicht vorauszusehen, daß die Verwendung von synthetischem Bienenwachs zur Erhöhung des Lichtschutzfaktors kosmetischer oder dermatologischer Zubereitungen, welche mindestens eine übliche UV-Filtersubstanz enthalten, den Nachteilen des Standes der Technik abhelfen würde.

Bienenwachs (Synonyme sind: Cera flava (gelblich) und Cera alba (weiß), oder nach CTFA: Beeswax) besteht hauptsächlich aus Myricylpalmitoat, Cerotinsäure, Melissinsäure, höheren Alkoholen und Kohlenwasserstoffen. Neuere Untersuchungen legen nahe, daß Bienenwachs aus einer homologen Reihe von vornehmlich Hexadecanoatalkylestern zwischen $C_{35}$ und $C_{54}$ bestehen ("Lexikon der Hilfsstoffe für Pharmazie, Kosmetik und angrenzende Gebiete", H.P. Fiedler, 3. Aufl., 1989, Editio Cantor Aulendorf, und dort unter dem Stichwort "Synthetisches Bienenwachs" zitierte Quellen und Querverweise).

Bienenwachs ist seit alters ein wichtiger kosmetischer Bestandteil, insbesondere für Cremes und Salbenzubereitungen, aber auch für kosmetische Stifte. Auch die Verwendung von Bienenwachsersatz in solchen Produkten ist bekannt.

Synthetisches Bienenwachs enthält nach R.B. Hutchison 45 - 70 Gew.-% hochmolekularer $\alpha$-verzweigte Monocarbonsäuren, 15 - 40 Gew.-% natürlicher oder synthetischer Gemischtglyceride gesättigter Mono- und/oder Dicarbonsäuren, z.B. Glycerinmonostearatmonoazelat, ferner 5 - 25 Gew.-% mikrokristallinen Petroleumwachs. Solches synthetisches Bienenwachs ist erhältlich gemäß der DE-OS 22 412 61 bzw. der US-PS 3 914 131.

Gleichwohl konnte der Stand der Technik nicht den Weg zu vorliegenden Erfindung ebnen.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% synthetischen Bienenwachses.

Erfindungsgemäße kosmetische und dermatologische Zubereitungen enthalten vorteilhaft, wenngleich nicht zwingend, anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Derlei Pigmente sind im Sinne der Erfindung als UV-Filtersubstanzen anzusehen, deren Beitrag zum Lichtschutzfaktor erfindungsgemäß durch Bienenwachs gesteigert wird.

Die anorganischen Pigmente liegen erfindungsgemäß vorteilhaft in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \text{ -> } n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure. Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Femer kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl,

Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglkolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß wird der LSF durch die Verwendung von synthetischem Bienenwachs in kosmetischen oder dermatologischen Zubereitungen, enthaltend übliche UVA-Filter und/oder UVB-Filter in der Lipidphase und/oder in der wäßrigen Phase erheblich gesteigert.

Insbesondere wird der LSF durch die Verwendung von synthetischem Bienenwachs in kosmetischen oder dermatologischen Zubereitungen, enthaltend Substanzen, die UV-Strahlung im UVB-Bereich absorbieren, wobei die Gesamtmenge solcher Filtersubstanzen z.B. 0,1 Gew.-% bis 30 Gew.-%, vorzugsweise 0,5 bis 20 Gew.-%, insbesondere 3 bis 15 Gew.-% beträgt, bezogen auf das Gesamtgewicht der Zubereitungen, erheblich gesteigert.

Solche UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;

Vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und deren Salze.

Die Liste der genannten UVB-Filter, die zusätzlich im Sinne der vorliegenden Erfindung eingesetzt werden

können, soll selbstverständlich nicht limitierend sein.

Insbesondere wird der LSF durch die Verwendung von synthetischem Bienenwachs in kosmetischen oder dermatologischen Zubereitungen, enthaltend Substanzen, die UV-Strahlung im UVA-Bereich absorbieren, wobei die Gesamtmenge solcher Filtersubstanzen z.B. 0,1 Gew.-% bis 30 Gew.-%, vorzugsweise 0,5 bis 20 Gew.-%, insbesondere 3 bis 15 Gew.-% beträgt, bezogen auf das Gesamtgewicht der Zubereitungen, erheblich gesteigert.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Insbesondere wird der LSF durch die Verwendung von synthetischem Bienenwachs in kosmetischen oder dermatologischen Zubereitungen, enthaltend polymergebundene oder polymere UV-Filtersubstanzen, wobei die Gesamtmenge solcher Filtersubstanzen z.B. 0,1 Gew.-% bis 30 Gew.-%, vorzugsweise 0,5 bis 20 Gew.-%, insbesondere 3 bis 15 Gew.-% beträgt, bezogen auf das Gesamtgewicht der Zubereitungen, erheblich gesteigert.

Die Gesamtmenge an wasserlöslichen UV-Filtersubstanzen in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Phenylbenzimidazol-5-sulfonsäure bzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Hydroxy-4-methoxybenzophenon-5-sulfonsäure bzw. deren Salzen in den fertigen kosmetischen oder dermetologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(2-Oxo-3-bornylidenmethyl)benzolsulfonsäure bzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Methyl-5-(2-oxo-3-bornylidenmethyl)benzolsulfonsäur bzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an Benzol-1,4-di(2-oxo-3-bomylidenmethyl-10-Sulfonsäurebzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4,4',4"-(1,3,5-Triazin-2,4,6-triyltriimino)-tris-benzoesäure-tris(2-ethylhexylester) in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(tert.-Butyl)-4'-methoxydibenzoylmethan in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-Methylbenzylidencampher in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt. bezogen auf das Gesamtgewicht der Zubereitungen.

Die Gesamtmenge an 2-Ethylhexyl-p-methoxy-cinnamat in den fertigen kosmetischen oder dermatologischen Zubereitungen wird, falls die Gegenwart dieser Substanz erwünscht ist, vorteilhaft aus dem Bereich von 0,1 - 15,0 Gew.-%, bevorzugt 0,5 - 7,5 Gew.-% gewählt, bezogen auf das Gesamtgewicht der Zubereitungen.

Noch eine weiterere erfindungsgemäß vorteilhaft zu verwendende zusätzliche Lichtschutzfiltersubstanz ist das Ethylhexyl-2-cyan-3,3-diphenylacrylat (Octocrylen), welches von BASF unter der Bezeichnung UVINUL® N 539 erhältlich ist und sich durch folgende Struktur auszeichnet:

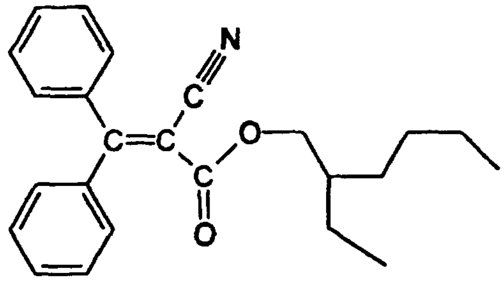

Die Gesamtmenge an Ethylhexyl-2-cyano-3,3-diphenylacrylat in den fertigen kosmetischen oder dermatologischen Zubereitungen wird, falls die Gegenwart dieser Substanz erwünscht ist, vorteilhaft aus dem Bereich von 0,1 - 15,0 Gew.-%, bevorzugt 0,5 - 10,0 Gew.-% gewählt, bezogen auf das Gesamtgewicht der Zubereitungen.
Ferner kann gegebenenfalls von Vorteil sein, erfindungsgemäß weiteren UVA- und/oder UVB-Filtern in kosmetische oder dermatologisch Zubereitungen einzuarbeiten, beispielsweise bestimmten Salicylsäurederivaten wie 4-Isopropylbenzylsalicylat, 2-Ethylhexylsalicylat (=Octylsalicylat), Homomenthylsalicylat.
Die Gesamtmenge an einem oder mehreren Salicylsäurederivaten in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 2,5 Gew.-% zu wählen. Wenn Homomenthylsalicylat gewählt wird, ist es von Vorteil, dessen Gesamtmenge aus dem Bereich von 0,1 - 10,0 Gew.-%, bevorzugt 0,5 - 5,0 Gew.-% zu wählen.
Der LSF kosmetischer oder dermatologischer Zubereitungen, enthaltend beliebige der vorab geschilderten UV-Filtersubstanzen, sei es als Einzelsubstanzen oder in beliebigen Gemischen untereinander, wobei die Gesamtmenge solcher Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, wird erfindungsgemäß durch die Verwendung von synthetischem Bienenwachs erheblich gesteigert.
Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispiel 1**

| O/W-Lotion | |
|---|---|
| | Gew.-% |
| Glycerylstearat SE | 3,50 |
| Stearinsäure | 1,80 |
| Glycerin | 3,00 |
| Cetearylalkohol | 0,50 |
| Octyldodecanol | 7,00 |
| Dicaprylylether | 8,00 |
| Uvinul®T150 | 3,00 |
| Synthetisches Bienenwachs | 1,00 |
| Carbomer | 0,20 |
| Natriumhydroxid (45%ig) | 0,20 |
| Konservierungsmittel | q.s. |
| Parfum | q.s. |
| Wasser, demin. | ad 100,00 |

**Beispiel 2**

| Hydrodispersionsgel | |
|---|---|
| | Gew.-% |
| Pemulen® TR-1 | 0,50 |
| Ethanol | 3,50 |
| Glycerin | 3,00 |
| Dimethicon | 1,50 |
| Octyldodecanol | 0,50 |
| Capryl-/Caprinsäuretriglycerid | 5,00 |
| Synthetisches Bienenwachs | 2,00 |
| Uvinul® T150 | 5,00 |
| Carbomer | 0,20 |
| Natriumhydroxid (45%ig) | 0,55 |
| Konservierungsmittel | q.s. |
| Parfum | q.s. |
| Wasser, demin. | ad 100,00 |

**Beispiel 3**

| O/W-Creme | |
|---|---|
| | Gew.-% |
| Glycerylstearat SE | 3,50 |
| Stearinsäure | 3,50 |
| Butylenglykol | 5,00 |
| Cetearylalkohol | 3,00 |
| $C_{12}$-$C_{15}$ Alkyl Benzoate | 10,0 |
| Uvinul T150 | 4,00 |
| Synthetisches Bienenwachs | 3,00 |
| Carbomer | 0,20 |
| Natriumhydroxid (45%ig) | 0,35 |
| Konservierungsmittel | q.s. |
| Parfum | q.s. |
| Wasser, demin. | ad 100,00 |

**Beispiel 4**

| W/O-Lotion | |
|---|---|
| | Gew.-% |
| Dehymuls® PGPH | 3,50 |
| Lameform® TGI | 3,50 |
| Butylenglykol | 5,00 |
| Ceresin | 3,00 |
| $C_{12}$-$C_{15}$ Alkyl Benzoate | 10,0 |
| Uvinul® T150 | 4,00 |

(fortgesetzt)

| W/O-Lotion | |
|---|---|
| | Gew.-% |
| Synthetisches Bienenwachs | 2,00 |
| Vaseline | 2,00 |
| Natriumhydroxid (45%ig) | 0,35 |
| Konservierungsmittel | q.s. |
| Parfum | q.s. |
| Wasser, demin. | ad 100,00 |

**Beispiel 5**

| O/W-Lotion | |
|---|---|
| | Gew.-% |
| Glycerylstearat SE | 3,50 |
| Stearinsäure | 1,80 |
| Glycerin | 3,00 |
| Cetearylalkohol | 0,50 |
| Octyldodecanol | 7,00 |
| Dicaprylylether | 8,00 |
| Uvinul®T150 | 3,00 |
| Synthetisches Bienenwachs | 1,00 |
| Uvinul® T150 | 4,00 |
| Parsol ®1789 | 2,00 |
| Eusolex® 6300 | 1,00 |
| Carbomer | 0,20 |
| Natriumhydroxid (45%ig) | 0,20 |
| Konservierungsmittel | q.s. |
| Parfum | q.s. |
| Wasser, demin. | ad 100,00 |

**Beispiel 6**

| Hydrodispersionsgel | |
|---|---|
| | Gew.-% |
| Pemulen® TR-1 | 0,50 |
| Ethanol | 3,50 |
| Glycerin | 3,00 |
| Dimethicon | 1,50 |
| Octyldodecanol | 0,50 |
| Capryl-/Caprinsäuretriglycerid | 5,00 |
| Synthetisches Bienenwachs | 2,00 |
| Uvinul® T150 | 5,00 |
| Uvinul® T150 | 4,00 |
| Parsol ®1789 | 2,00 |
| Eusolex® 6300 | 1,00 |

(fortgesetzt)

| Hydrodispersionsgel | |
|---|---|
| | Gew.-% |
| Carbomer | 0,20 |
| Natriumhydroxid (45%ig) | 0,55 |
| Konservierungsmittel | q.s. |
| Parfum | q.s. |
| Wasser, demin. | ad 100,00 |

**Beispiel 7**

| O/W-Creme | |
|---|---|
| | Gew.-% |
| Glycerylstearat SE | 3,50 |
| Stearinsäure | 3,50 |
| Butylenglykol | 5,00 |
| Cetearylalkohol | 3,00 |
| $C_{12}$-$C_{15}$ Alkyl Benzoate | 10,0 |
| Uvinul T150 | 4,00 |
| Uvinul® T150 | 4,00 |
| Parsol ®1789 | 2,00 |
| Eusolex® 6300 | 1,00 |
| Synthetisches Bienenwachs | 3,00 |
| Carbomer | 0,20 |
| Natriumhydroxid (45%ig) | 0,35 |
| Konservierungsmittel | q.s. |
| Parfum | q.s. |
| Wasser, demin. | ad 100,00 |

**Beispiel 8**

| W/O-Lotion | |
|---|---|
| | Gew.-% |
| Dehymuls® PGPH | 3,50 |
| Lameform® TGI | 3,50 |
| Butylenglykol | 5,00 |
| Ceresin | 3,00 |
| $C_{12}$-$C_{15}$ Alkyl Benzoate | 10,0 |
| Uvinul® T150 | 4,00 |
| Parsol ®1789 | 2,00 |
| Eusolex® 6300 | 1,00 |
| Titandioxid | 2,00 |
| Synthetisches Bienenwachs | 2,00 |
| Vaseline | 2,00 |
| Natriumhydroxid (45%ig) | 0,35 |
| Konservierungsmittel | q.s. |

(fortgesetzt)

| W/O-Lotion | |
|---|---|
| | Gew.-% |
| Parfum | q.s. |
| Wasser, demin. | ad 100,00 |

**Patentansprüche**

1. Verwendung von synthetischem Bienenwachs zur Erhöhung des Lichtschutzfaktors kosmetischer oder dermatologischer Zubereitungen, welche mindestens eine übliche UV-Filtersubstanz enthalten.

2. Verwendung nach Anspruch 1, **dadurch gekennzeichnet, daß** die kosmetischen oder dermatologischen Lichtschutzzubereitungen 0,1 bis 20 Gew.-% synthetischen Bienenwachses enthalten.

3. Verwendung nach Anspruch 1, **dadurch gekennzeichnet, daß** die kosmetischen oder dermatologischen Lichtschutzzubereitungen 0,5 bis 10 Gew.-% synthetischen Bienenwachses enthalten.

4. Verwendung nach Anspruch 1, **dadurch gekennzeichnet, daß** die kosmetischen oder dermatologischen Lichtschutzzubereitungen 1 bis 5 Gew.-% synthetischen Bienenwachses enthalten.

5. Verwendung nach Anspruch 1, **dadurch gekennzeichnet, daß** das synthetische Bienenwachs

   - 45 - 70 Gew.-% hochmolekularer $\alpha$-verzweigte Monocarbonsäuren,
   - 15 - 40 Gew.% natürlicher oder synthetischer Gemischtglyceride gesättigter Monound/oder Dicarbonsäuren, z.B. Glycerinmonostearatmonoazelat,
   - 5 - 25 Gew.-% mikrokristallinen Petroleumwachses.

   enthalten.

6. Verwendung nach Anspruch 1, **dadurch gekennzeichnet, daß** die kosmetischen oder dermatologischen Lichtschutzzubereitungen als UV-Filtersubstanz den 4,4',4"-(1,3,5-Triazin-2,4,6-triyltriimino)-tris-benzoesäure-tris (2-ethylhexylester) enthalten.

**Claims**

1. Use of synthetic beeswax for increasing the light protection factor of cosmetic or dermatological preparations which comprise at least one customary UV filter substance.

2. Use according to Claim 1, **characterized in that** the cosmetic or dermatological light protection preparations comprise 0.1 to 20% by weight of synthetic beeswax.

3. Use according to Claim 1, **characterized in that** the cosmetic or dermatological light protection preparations comprise 0.5 to 10% by weight of synthetic beeswax.

4. Use according to Claim 1, **characterized in that** the cosmetic or dermatological light protection preparations comprise 1 to 5% by weight of synthetic beeswax.

5. Use according to Claim 1, **characterized in that** the synthetic beeswax comprises

   - 45-70% by weight of high molecular weight $\alpha$-branched monocarboxylic acids,
   - 15-40% by weight of natural or synthetic mixed glycerides of saturated mono- and/or dicarboxylic acids, e.g. glycerol monostearate monoazelate,
   - 5-25% by weight of microcrystalline petroleum wax.

**6.** Use according to Claim 1, **characterized in that** the cosmetic or dermatological light protection preparations comprise tris(2-ethylhexyl) 4,4',4"-(1,3,5-triazine-2,4,6-triyltriimino)trisbenzoate as UV filter substance.

## Revendications

**1.** Utilisation de cire d'abeilles synthétique en vue de l'augmentation du facteur de protection solairede préparations cosmétiques ou dermatologiques, qui contiennent au moins une substance de filtre UV usuelle.

**2.** Utilisation selon la revendication 1, **caractérisée en ce que** les préparations de protection solairecosmétiques ou dermatologiques contiennent de 0,1 à 20% en poids de cire d'abeilles synthétique.

**3.** Utilisation selon la revendication 1, **caractérisée en ce que** les préparations de protection solairecosmétiques ou dermatologiques contiennent de 0,1 à 10% en poids de cire d'abeilles synthétique.

**4.** Utilisation selon la revendication 1, **caractérisée en ce que** les préparations de protection solairecosmétiques ou dermatologiques contiennent de 1 à 5% en poids de cire d'abeilles synthétique.

**5.** Utilisation selon la revendication 1, **caractérisée en ce que** la cire d'abeilles synthétique contient

- de 45 - 70% en poids d'acides monocarboxyliques $\alpha$-ramifiés à poids moléculaire élevé,
- de 15 - 40% en poids de glycérides mixtes, naturelles ou synthétiques, d'acides monocarboxyliques et/ou dicarboxyliques saturés, par exemple, le monostéarate-monoazélate de glycérine,
- 5 - 25% en poids de cire de pétrole microcristalline.

**6.** Utilisation selon la revendication 1, **caractérisée en ce que** les préparations de protection contre la lumière cosmétiques ou dermatologiques contiennent, en tant que substance de filtre UV, l'ester tri(2-éthylhexylique) de l'acide 4,4',4"-(1,3,5-triazin-2,4,6-triyltriimino)-tris-benzoique.